(19) Europäisches Patentamt
European Patent Office
Office européen des brevets

(11) Veröffentlichungsnummer: **0 177 450**
**B1**

(12) **EUROPÄISCHE PATENTSCHRIFT**

(45) Veröffentlichungstag der Patentschrift: **29.03.89**

(21) Anmeldenummer: **85810441.7**

(22) Anmeldetag: **26.09.85**

(51) Int. Cl.⁴: **C 07 C 62/00,** C 07 C 51/347, C 07 C 69/757, C 07 C 67/30, C 07 C 93/197, C 07 C 89/00, C 07 C 103/19, C 07 C 103/737, C 07 C 102/00, C 07 C 149/14, C 07 C 149/26

(54) **Verfahren zur Herstellung von Cyclohexandion-carbonsäurederivaten mit herbizider und das Pflanzenwachstum regulierender Wirkung.**

(30) Priorität: **02.10.84 CH 4734/84**

(43) Veröffentlichungstag der Anmeldung: **09.04.86 Patentblatt 86/15**

(45) Bekanntmachung des Hinweises auf die Patenterteilung: **29.03.89 Patentblatt 89/13**

(84) Benannte Vertragsstaaten: **BE CH DE FR GB IT LI NL**

(56) Entgegenhaltungen:
**EP-A-0 090 262**
**EP-A-0 123 001**
**EP-A-0 126 713**

(73) Patentinhaber: **CIBA- GEIGY AG, Klybeckstrasse 141, CH- 4002 Basel (CH)**

(72) Erfinder: **Brunner, Hans- Georg, Dr., Wannenstrasse 14, CH- 4415 Lausen (CH)**
Erfinder: **Müller, Urs, Dr., Drosselstrasse 6, CH- 4142 Münchenstein (CH)**

**EP 0 177 450 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft ein neues, optimiertes Verfahren zur Herstellung von Cyclohexandion-carbonsäurederivaten mit herbizider und das Pflanzenwachstum regulierender Wirkung. Es werden neue Produkte offenbart sowie Mittel welche diese Cyclohexandion-carbonsäurederivate enthalten und deren Verwendung zur Bekämpfung von Unkräutern oder zur Regulierung des Pflanzenwachstums.

Bei den Cyclohexandion-carbonsäurederivaten handelt es sich um 4-Acyl-3,5-cyclohexandion-1-carbonsäurederivate, welche der Formel I entsprechen

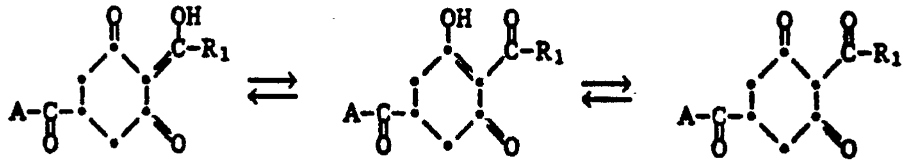

(I)

worin

A einen Rest $-OR_2$ oder $-NR_3R_4$ oder ein Metall- oder Ammoniumsalz davon,

$R_1$ $C_1$-$C_6$ Alkyl oder $C_3$-$C_6$ Cycloalkyl, unsubstituiert oder substituiert durch Halogen, $C_1$-$C_4$ Alkoxy oder $C_1$-$C_4$ Alkylthio,

$R_2$, $R_3$ und $R_4$ unabhängig voneinander je Wasserstoff; $C_1$-$C_6$ Alkyl, $C_1$-$C_4$ Halogenalkyl, $C_2$-$C_{10}$ Alkoxyalkyl, $C_2$-$C_{10}$ Alkylthioalkyl; $C_3$-$C_6$ Alkenyl, unsubstituiert oder substituiert durch Halogen, $C_1$-$C_4$ Alkoxy oder $C_1$-$C_4$ Alkylthio; $C_3$-$C_6$ Alkinyl oder Phenyl, wobei der Phenylkern unsubstituiert oder durch Halogen, $C_1$-$C_4$ Alkyl, $C_1$-$C_4$ Alkoxy, $C_1$-$C_4$ Halogenalkyl, Nitro oder Cyan substituiert ist,

$R_3$ und $R_4$ zusammen mit dem Stickstoffatom, an das sie gebunden sind, bilden auch einen 5 - 6 gliedrigen Heterocyclus, der im Ring noch ein Sauerstoff- oder Schwefelatom enthalten kann,

bedeuten.

In diesen Definitionen umfassen die Alkylreste zowohl geradkettige wie verzweigte, z. B. Methyl, Äthyl, Propyl, Isopropyl, Butyl, Isobutyl, sec.-Butyl, tert.Butyl sowie alle stereoisomeren Formen der höheren Homologen. Ebenso werden unter Alkenyl und Alkinyl geradkettige wie verzweigte Reste verstanden, z. B. Vinyl, Allyl, Methallyl, Butenyl, Methyl- und Dimethylbutenyl, Äthinyl, Propinyl, Butinyl, Methylbutinyl, Dimethylbutinyl.

Unter Halogen ist Fluor-, Chlor-, Brom- oder Jodatom zu verstehen.

Unter den von $R_3$ und R4 zusammen mit dem Stickstoffatom gebildeten 5 - 6 gliedrigen Hetercyclen, welche im Ring noch ein Sauerstoff- oder Schwefelatom enthalten können, sind Pyrrol, Pyrrolidin, Piperidin, Morpholin oder auch Thiomorpholin-Reste zu verstehen. Solche Ringe können durch Methylgruppen substituiert sein.

Die Salze dieser Verbindungen werden mit Basen hergestellt. Dazu eignen sich vorzugsweise Alkalimetall-, Erdalkalimetall-, Eisen-, Kupfer-, Nickel-, Zinkhydroxide, aber auch Ammoniak, oder quaternäre $C_1$-$C_4$ Alkyl- oder $C_1$-$C_4$ Hydroxyalkylammonium-Basen.

Die Cyclohexandioncarbonsäurederivate der Formel I zeichnen sich durch gute herbizide und den Pflanzenwuchs regulierende Wirkung aus. Sie sind unter Normalbedingungen feste oder zähflüssige Verbindungen, die sich ohne besondere Vorsichtsmassnahmen manipulieren lassen.

In der Literatur sind Verfahren beschrieben worden, gemäss denen solche und ähnliche Verbindungen hergestellt werden oder hergestellt werden könnten. In all diesen Verfahren müssen Zwischenprodukte isoliert und wieder in die Reaktion geben werden, was die Arbeitsabläufe kompliziert und die Ausbeute drückt.

In der Japanischen Offenlegungsschrift JA-A 58-164.543 z. B. werden solche Verbindungen in 60 - 72 %-iger Ausbeute hergestellt, indem man zu einem 3,5-Cyclohexandion-1-carbonsäureester ein Säurechlorid gibt, den entstandenen 3-Acyloxy-cyclohex-3-en-5-oncarbonsäureester ein Säurechlorid gibt, den entstandenen 3-Acyloxy-cyclohex-3-en-5-oncarbonsäureester dann durch mehrstündiges Kochen in Toluol in Gegenwart einer katalytischen Menge von Dimethylaminopyridin (γ-Picolin) 4-Pyrrolidinoaminopyridin, N-Methylimidiazol etc.) zum gewünschten 4-Acyl-3,5-cyclohexandion-1-carbonsäurederivat umwandelt.

In der offengelegten Europäischen Patentanmeldung EP-A-90 262 wird ein Verfahren zur Herstellung von 2-Benzoyl-1,3-cyclohexandionen-Verbindungen beschrieben, gemäss dem 1,3-Cyclohexandion mit Benzoylcyanid in Gegenwart von Zinkchlorid und einer organischen Base zusammengegenen werden. Das Kondensationsprodukt wird isoliert, in Äther aufgelöst und mit Kupferacetat in ein Kupferkomplexsalz überführt, das aus dem Reaktionsgemisch durch Filtration isoliert wird bevor man es in Salzsäure auflöst und aus der sauren Lösung durch Extraktion das gewünschte 2-Acyl-1,3-cyclohexandion isoliert werden kann. Auch bei diesem Herstellungsverfahren, dessen Ausbeute mit 78 % angegeben wird, werden zweimal Zwischenprodukte isoliert. Dabei ist die Herstellung des als Ausgangsmaterial benötigten Benzoylcyanides gemäss Org. Synthesis Coll. Vol. III (1955) p. 122, wobei das Benzoylchlorid unter gegebenen Vorsichtsmassnahmen mit einem Metallcyanid vereinigt wird, nicht berücksichtigt.

2-Acyl-1,3-cyclohexandion-Verbindungen lassen sich gemäss Synthesis 925 (1978) auch herstellen, indem man in einer ersten Stufe das 1,3-Cyclohexandion in Gegenwart eines inerten organischen Lösungsmittels und einer organischen Base mit einem Säurechlorid kondensiert. Das entstandene Acyloxy-cyclohex-3-en-3-on wird isoliert und in einer zweiten Reaktionsstufe in Gegenwart einer Lewis-säure wie z. B. Aluminiumtrichlorid zur

gewünschten 2-Acyl-3,5-cyclohexandion-Verbindung umzulagern.

Obwohl diese durch Lewis-Säuren katalysierte C-Acylierung bekannt war, konnte sie bisher nicht mit Erfolg für die Herstellung der erfindungsgemässen 2-Acyl-3,5-cyclohexandion-1-carbonsäureester angewandt resp. modifiziert werden.

Die Cyclohexandion-carbonsäurederivate der Formel I lassen sich gemäss einem neuen optimierten Verfahren in einem einzigen Reaktionsvorgang in ausgezeichneter Ausbeute herstellen. Man gibt dabei in einem Reaktionsgefäss ein in einem inerten Lösungsmittel gelöstes Säurehalogenid und ein Metallcyanid vorzugsweise ein Kupfer- oder Alkalimetallcyanid vor, zu welchem man nach kurzem Aufheizen bei 0° bis Raumtemperatur nacheinander Zinkchlorid, ein 3,5-Cyclohexandion-1-carbonsäurederivat, und schliesslich tropfenweise eine organische Base zugibt. Das Reaktionsgemisch wird dann mit wässriger Säure angesäuert und die extrahierte und vom Lösungsmittel befreite organische Phase ist bereits das gewünschte 4-Acyl-1,3-cyclohexandion-1-carbonsäurederivat der Formel I. Das Verfahren kann als Eintopfverfahren bezeichnet werden, da bei diesen Operation weder Zwischenprodukte isoliert noch das Reaktionsgefäss gewechselt werden muss.

Die Reaktionsfolge kann mit folgendem Formelschema angedeutet werden:

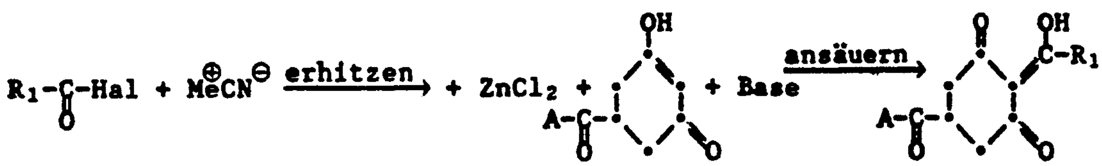


II I

Als Zwischenprodukt muss dabei zuerst Säurecyanid der Formel

$R_1$-C-CN
‖
O

entstehen, welches mit dem 3,5-Cyclohexandion-1-carbonsäurederivat der Formel II reagiert.

Das neue erfindungsgemässe Verfahren ermöglich nun in einer einzigen Reaktionsfolge, in praktisch quantitativer Ausbeute ohne dabei Zwischenprodukte isolieren zu müssen, die 4-Acyl-3,5-cyclohexandion-1-carbonsäurederivate der Formel I herzustellen.

Das erfindungsgemässe Verfahren zur Herstellung der Cyclohexandioncarbonsäurederivate der Formel I ist dadurch gekennzeichnet, dass man in einem inerten organischen Lösungsmittel ein reaktionsfähiges Carbonsäurederivat der Formel III

X - C - $R_1$ (III),
‖
O

worin X ein Halogenatom, einen Alkyl- oder Arylsulfonsäurerest oder der zum Anhydrid fehlende Molekularrest

-O-C-$R_1$
‖
O

bedeutet, während $R_1$ die unter der Formel I gegebene Bedeutung hat, und ein Metallcyanid zusammengibt, das Reaktionsgemisch zum Sieden erhitzt und nach Abkühlen auf einen Temperaturbereich von 0°C bis Raumtemperatur, dazu nacheinander eine Lewis-Säure, die zum Carbonsäurederivat der Formel III äquimolare Menge eines 3,5-Cyclohexandion-1-carbonsäurederivates der Formel II

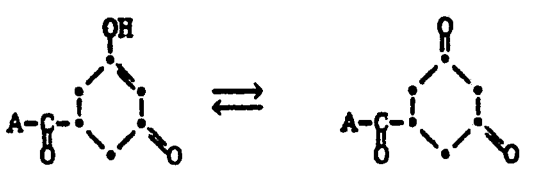

(II)

worin A die unter der Formel I gegebene Bedeutung hat und tropfenweise eine organische Base gibt und nach Ansäuern des Reaktionsgemisches mit einer wässrigen Säure daraus das 4-Acyl-3,5-cyclohexandion-1-carbonsäurederivat der Formel I durch Extraktion isoliert.

Als inerte organische Lösungsmittel für dieses Verfahren eignen sich vor allem Acetonitril, die halogenierten

Kohlenwasserstoffe wie z. B. Chloroform, Methylenchlorid, Äthylenchlorid, (1,2-Dichloräthan), ferner höhersiedende Äther wie Diisopropyläther, Dioxan oder Tetrahydrofuran, auch höhersiedende Ketone wie Methyläthylketon, zudem Dimethylformamid, Dimethylsulfoxid.

Als reaktionsfähiges Säurederivat der Formel III wird vorzugsweise ein Halogenid, wie das Chlor oder Bromid, verwendet.

Die in dieser Reaktion bevorzugt verwendeten Metallcyanide sind das Kupfer-, Kalium- oder Natriumcyanid oder Mischungen derselben.

Es ist vorteilhaft, wenn man die Mischung des reaktionsfähigen Carbonsäurederivates der Formel III mit dem Metallcyanid im inerten Lösungsmittel zuerst während 1 bis 25 stunden am Rückfluss erhitzt ehe man abkühlt und die Reaktion bei einem Temperaturbereich von 0°C bis Raumtemperatur fortführt.

Als Lewis-Säure wird in dieser Reaktion vorzugsweise Zinkchlorid verwendet, es können jedoch auch Aluminiumchlorid, Antimonchlorid und andere verwendet werden.

Die folgenden organischen Basen können dem Reaktionsgemisch zugetropft werden: Trimethylamin, Diäthylamin, Triäthylamin, γ-Picolin, 4-N,N-Dimethylpyridin, 4-N,N-Diäthylpyridin, 4-Pyrolidinopyridin, N-Methylimidazol oder 4-Äthylimidazol. Es kommen noch weitere organische und auch anorganische Basen für diesen Zweck in Frage, jedoch haben sich in der Praxis die obigen für einen ruhigen und vollständigen Reaktionsablauf am besten bewährt.

Zum Abschluss wird das Reaktionsgemisch mit einer wässrigen Säure angesäuert. Dabei hat sich das Gemisch 6N Salzsäure/Eis am besten bewährt. Es entstehen zwei Phasen, wobei die organische Phase im wesentlichen aus dem 4-Acyl-3,5-cyclohexan-dion-1-carbonsäurederivat der Formel I und Lösungsmittel besteht, während die Salze und gegebenenfalls nicht reagierte Carbonsäure oder Derivate der Formel III in der wässrigen Phase verbleiben und verworfen werden.

Viele Verbindungen der Formel I sind bekannt und z. B. in der Japanischen offengelegten Patentanmeldung JA-A 58-164 543 beschrieben, andere hingegen sind neu und werden zum erstenmal hergestellt. Die Erfindung umfasst auch diese neuen Verbindungen. Neu sind diejenigen 4-Acyl-3,5-cyclohexan-dion-1-carbonsäurederivate der Formel I, während A einen unter der Formel I definierten Ester- oder Amidrest darstellt.

Zum erstenmal hergestellt wurden die folgenden Verbindungen: 4-Cyclopropylcarbonyl-3,5-cyclohexandion-1-carbonsäure sowie deren Methyl-, und Äthylester
4-Trimethylacetyl-3,5-cyclohexandion-1-carbonsäure-äthylester,
4-Cyclobutylcarbonyl-3,5-cyclohexandion-1-carbonsäure-dimethylamid,
4-Butyryl-3,5-cyclohexandion-1-carbonsäuremethylthiopropylamid,
4-Butyryl-3,5-cyclohexandion-1-carbonsäure-diallylamid,
4-Butyryl-3,5-cyclohexandion-1-carbonsäure-pyrrolidonamid,
4-Propionyl-3,5-cyclohexandion-1-carbonsäure-dimethylamid
4-Hexamoyl-3,5-cyclohexandion-1-carbonsäure-dimethylamid.

Die als Ausgangsstoff benötigten Cyclohexandioncarbonsäurederivate der Formel II werden einerseits durch Hydrieren von 3,5-Dihydroxybenzoesäure mit Wasserstoff und Raney-Nickel und nachheriger Veresterung oder Amidierung des Säurerestes entsprechend dem folgenden Schema erhalten

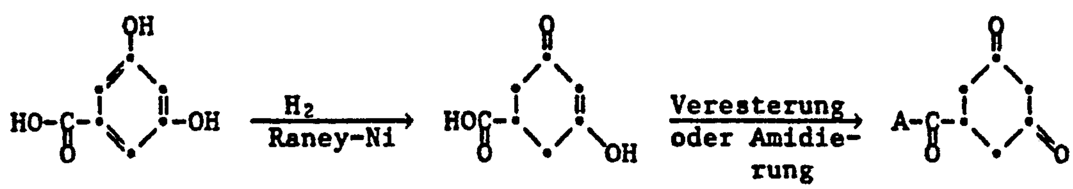


wobei die Ketogruppe gegebenenfalls geschützt werden muss, z. B. als Enoläther oder als Enamin, siehe dazu auch J. Am., Chem. Soc. 78, 4405 (1956).

Man kann aber auch ein 3,5-Dihydroxybenzoesäurederivat mit Wasserstoff und Raney-Nickel hydrieren entsprechend dem Schema

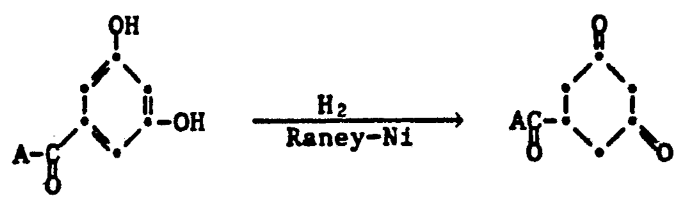


siehe Arch. Pharm. 307, 577 (1974).

Bei geringen Aufwandmengen zeichnen sich die Verbindungen der Formel I durch gute selektivewuchshemmende und selektiv-herbizide Eigenschaften aus, die sie ausgezeichnet zum Einsatz in Kulturen von Nutzpflanzen, insbesondere in Zuckerrohr, Getreide, Baumwolle, Soja, Mais und Reis befähigen.

Die Wirkungsart dieser Wirkstoffe ist unüblich. Viele sind translozierbar, d. h. sie werden von der Pflanze aufgenommen und an andere Stellen transportiert, wo sie dann zur Wirkung kommen.

Die Verbindungen der Formel I haben ausserdem starke pflanzenwuchsregulierende Eigenschaften, die sich in einer Ertragssteigerung von Kulturpflanzen oder Erntegut auswirken kann. Viele Verbindungen der Formel I zeigen darüberhinaus eine konzentrationsabhängige pflanzenwuchshemmende Wirkung. Es werden sowohl Monokotyledonen als auch Dikotyledonen in ihrem Wachstum beeinträchtigt.

So können z. B. die in der Landwirtschaft in tropischen Gegenden häufig als "cover crops" (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Bei monokotylen Pflanzen, z. B. Gräsern oder auch Kulturpflanzen wie Getreide, ist eine Hemmung des vegetativen Wachstums manchmal gewünscht und vorteilhaft. Eine derartige Wuchshemmung ist unter anderem bei Gräsern von wirtschaftlichem Interesse, denn dadurch kann z. B. die Häufigkeit der Grasschnitte in Ziergärten, Park- und Sportanlagen oder an Strassenrändern reduziert werden. Von Bedeutung ist auch die Hemmung des Wuchses von krautigen und holzigen Pflanzen an Strassenrändern und in der Nähe von Überlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens ("Lagerns") der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt.

Bei grösseren Aufwandmengen werden alle getesteten Pflanzen in ihrer Entwicklung so geschädigt, dass sie absterben.

Die Erfindung betrifft auch herbizide und pflanzenwuchsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, durch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegegebenenfalls epoxidiertes Pflanzenöl wie epoxydiertes Kokosnussöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückständ, verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$ - $C_{22}$), wie z. B. die Na- oder K-Salze der Öl- oder Stearinsäure, oder von natürlichen Fettsäuregemischen die z. B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2 Sulfonsäuregruppen und einen Fettsäurerest mit 8 - 22 C-Atomen. Alkylarylsulfonate sind z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Äthylenoxid-Adduktes in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Oxtylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die salze liegen vorzugsweise als Halogenide, Methylsulfate oder Äthylsulfate vor, z. B. das Stearyltrimethylammoniumchlorid oder das Benzyldi(2-chloräthyl)-äthyl-ammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1979.

M. and J. Ash, "Encyclopedia of Surfactants" Vol. I - III, Chemical Publishing Co., Inc. New York, 1981.

H. Stache "Tensid-Taschenbuch", C. Hanser Verlag, München und Wien 1982.

Die Wirkstoffzubereitungen enthalten in der Regel 0,1 bis 99 %, insbesondere 0,1 bis 80 %, Wirkstoff der Formel I, 1 bis 99 % eines festen oder flüssigen Zusatzstoffes und 0 bis 25 %, insbesonders 0,1 bis 25 %, eines Tensides.

Insbesondere setzensich bevorzugte Formulierungen folgendermassen zusammen: (% = Gewichtsprozent)

| | | |
|---|---|---|
| Emulgierbare Konzentrate | | |
| Aktiver Wirkstoff: | 1 bis 50 %, bevorzugt | 5 bis 10 % |
| oberflächenaktives Mittel: | 5 bis 30 %, vorzugsweise | 10 bis 20 % |
| flüssiges Trägermittel: | 50 bis 94 %, vorzugsweise | 70 bis 85 %. |
| Stäube | | |
| Aktiver Wirkstoff: | 0,1 bis 10 %, vorzugsweise | 0,1 bis 1 % |
| festes Trägermaterial: | 99,9 bis 90 %, vorzugsweise | 99,9 bis 99 %. |
| Suspensions-Konzentrat | | |
| Aktiver Wirkstoff: | 5 bis 75 %, vorzugsweise | 10 bis 50 % |
| Wasser: | 94 bis 25 %, vorzugsweise | 90 bis 30 % |
| oberflächenaktives Mittel: | 1 bis 40 %, vorzugsweise | 2 bis 30 %. |
| Benetzbare Pulver | | |
| Aktiver Wirkstoff: | 0,5 bis 90 %, vorzugsweise | 1 bis 80 % |
| oberflächenaktives Mittel: | 0,5 bis 20 %, vorzugsweise | 1 bis 15 % |
| festes Trägermaterial: | 5 bis 95 %, vorzugsweise | 15 bis 90 %. |
| Granulate | | |
| Aktiver Wirkstoff: | 0,5 bis 30 %, vorzugsweise | 3 bis 15 % |
| festes Trägermittel: | 99,5 bis 70 %, vorzugsweise | 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden.

Die Aufwandmengen betragen in der Regel 0,01 bis 10 kg/ha, vorzugsweise 0,025 bis 5 kg/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C, die Drücke in Millibar mbar angegeben.

**Herstellungsbeispiel:**

**Beispiel 1:**

Herstellung von 4-Cyclopropylcarbonyl-3,5-cyclohexandion-1-carbonsäure-äthylester

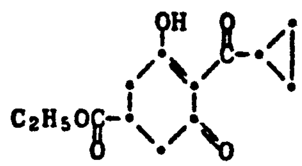

5,4 g Kupfercyanid werden in 40 ml Acetonitril vorgelegt und dazu tropft man bei Raumtemperatur 5,1 ml Cyclopropylcarbonsäurechlorid. Die Reaktionstemperatur steigt dabei auf 31°. Nachdem alles zugegeben ist, kocht man das Reaktionsgemisch am Rückfluss unter Rühren während 2 1/2 Stunden. Anschliessend wird auf Raumtemperatur abgekühlt und zum Reaktionsgemisch 9,2 g 3 5-Cyclohexandion-1-carbonsäureäthylester und 7,1 g Zinkchlorid gegeben. Dazu wird anschliessend bei einer Temperatur von 0 - 5° eine Lösung von 7,4 ml Triäthylamin in 10 ml Acetonitril getropft. Man lässt das Reaktionsgemisch während ca. 14 Stunden (über Nacht) bei Raumtemperatur weiterrühren und versetzt es dann mit 100 ml kalter 4N Salzsäure. Das Gemisch wird dann zweimal mit je 200 ml Chloroform extrahiert, die organische Phase über Natriumsulfat getrocknet, eingedampft und der Rückstand am Hochvakuum (Kugelrohrofen) destilliert. Ein zähflüssiges Öl destilliert bei 150°/0,005 mbar und kristallisiert beim Stehen. Ausbeute 7,9 g kristalliner 4-Cyclopropyloxy-3,5-cyclohexandion-1-carbonsäure-äthylester, Smp. 35 - 36° (Ausbeute 62,7 % der Theorie).

Durch Aufkochen dieses Esters in 1N Natronlauge und Extrahieren der erkalteten Lösung erhält man die 4-Cyclopropyloxy-3,5-cyclohexandion-1-carbonsäure, welche bei 142 - 146° schmilzt.

Anstelle von Kupfercyanid kann in diesem Beispiel auch Natriumcyanid oder Cyankali verwendet werden.

In analoger Weise zu diesem Beispiel werden folgende Verbindungen erhalten.

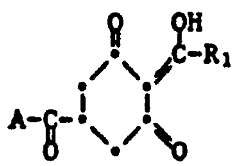

**Tabelle 1** A = $OR_2$

| No. | A | $R_1$ | phys. Daten |
|---|---|---|---|
| 1.01 | $OC_2H$ | $CH_3$ | Smp. 49 - 51° |
| 1.02 | $OC_3H_7n$ | $CH_3$ | Smp. 43 - 44° |
| 1.03 | $OCH(CH_3)_2$ | $CH_3$ | Smp. 98 - 100° |
| 1.04 | $OC_4H_9n$ | $CH_3$ | $n_D^{20}$ 1.4991 |
| 1.05 | $OCH_2CH(CH_3)_2$ | $CH_3$ | Smp. 39 - 40° |
| 1.06 | $OC(CH_3)_3$ | $CH_3$ | Smp. 76 - 77° |
| 1.07 | $OCH(CH_3)C_2H_5$ | $CH_3$ | Smp. 36 - 37° |
| 1.08 | $OCH_3$ | $C_2H_5$ | Smp. 49 - 50° |
| 1.09 | $OC_2H_5$ | $C_2H_5$ | Smp. 51 - 52° |
| 1.10 | $OC_3H_7$ | $C_2H_5$ | $n_D^{20}$ 1.4960 |
| 1.11 | $OCH(CH_3)_2$ | $C_2H_5$ | Smp. 55 - 56° |
| 1.12 | OH | $C_2H_5$ | Smp. 98 - 99° |
| 1.13 | OH | $C_3H_7n$ | Smp. 120 - 135° |
| 1.14 | $OCH_3$ | $C_3H_7n$ | $n_D^{20}$ 1.5685 |
| 1.15 | $OC_2H_5$ | $C_3H_7n$ | $n_D^{27}$ 1.5060 |
| 1.16 | $OC_3H_7n$ | $C_3H_7n$ | $n_D^{20}$ 1.5020 |
| 1.17 | $OCH(CH_3)_2$ | $C_3H_7n$ | $n_D^{25}$ 1.4998 |

| | | | |
|---|---|---|---|
| 1.18 | $OC_4H_9n$ | $C_3H_7n$ | $n_D^{20}$ 1.4930 |
| 1.19 | $OCH_2CN(CH_3)_2$ | $C_3H_7n$ | $n_D^{20}$ 1.4907 |
| 1.20 | $OCH(CH_3)C_2H_5$ | $C_3H_7n$ | $n_D^{20}$ 1.4893 |
| 1.21 | $OCH(C_2H_5)_2$ | $C_3H_7n$ | $n_D^{20}$ 1.4885 |
| 1.22 | OH | $CH(CH_3)_2$ | Smp. 132° |
| 1.23 | $OCH_3$ | $CH(CH_3)_2$ | Sdp. 110°/0.01 mbar |
| 1.24 | $OC_2H_5$ | $CH(CH_3)_2$ | $n_D^{20}$ 1.4898 |
| 1.25 | $OC_2H_5$ | $C_4H_9n$ | $n_D^{20}$ 1.4943 |
| 1.26 | $OC_2H_5$ | $CH_2CH(CH_3)_2$ | $n_D^{20}$ 1.4948 |
| 1.27 | $OC_2H_5$ | $C_5H_{11}n$ | $n_D^{20}$ 1.4803 |
| 1.28 | $OC_2H_5$ | $C_6H_{13}n$ | $n_D^{20}$ 1.4803 |
| 1.29 | $OC_2H_5$ | $C_7H_{15}n$ | $n_D^{20}$ 1.4773 |
| 1.30 | $OC_2H_5$ | $C_8H_{17}n$ | $n_D^{20}$ 1.4650 |
| 1.31 | $OC_2H_5$ | $C_2H_4Cl$ | |
| 1.32 | $OC_2H_5$ | $C_3H_6Cl$ | |
| 1.33 | $OC_2H_5$ | $C_4H_9Cl$ | |
| 1.34 | $OC_2H_5$ | $CH_2OC_2H_5$ | $n_D^{20}$ 1.5035 |
| 1.35 | $OC_2H_5$ | $CH_2SC_2H_5$ | $n_D^{20}$ 1.5357 |
| 1.36 | OH | $CH_3$ | Smp. 139 - 141° |
| 1.37 | $OCH_3$ | $CH_3$ | Smp. 77 - 78° |
| 1.38 | $OC(CH_3)$ | $C_4H_9n$ | Smp. 67 - 69° |
| 1.39 | $OC_2H_4SCH_3$ | $C_3H_7n$ | $n_D^{20}$ 1.5170 |
| 1.40 | $OC_2H_5$ | $C_2H_4OCH_3$ | |
| 1.41 | $OC_2H_5$ | $C_2H_4OC_2H_5$ | |
| 1.42 | $OC_2H_5$ | $CH_2OC_3H_7n$ | |
| 1.43 | $OC_2H_5$ | $C_2H_4SCH_3$ | |
| 1.44 | $OC_2H_5$ | $CH_2SCH_3$ | |
| 1.45 | OH | $C_2H_4SC_3H_7n$ | |
| 1.46 | $OCH_2SCH_3$ | $C_3H_7n$ | $n_D^{25}$ 1.5193 |
| 1.47 | $OC_2H_4SCH_3$ | $C_3H_7n$ | |
| 1.48 | $OC_2H_4OCH_3$ | $C_3H_7n$ | |
| 1.49 | $OC_3H_7i$ | $C_3H_7n$ | |
| 1.50 | $OC_3H_6Cl$ | $C_3H_7n$ | $n_D^{25}$ 1.4998 |
| 1.51 | Benzyloxy | $C_3H_7n$ | |
| 1.52 | 3-Methoxybenzyloxy | $C_3H_7n$ | |
| 1.53 | $OCH_2CH=CH_2$ | $C_3H_7n$ | |
| 1.54 | $OCH_2CCl=CH_2$ | $C_3H_7n$ | |
| 1.55 | $OCH_2C\equiv CH$ | $C_3H_7n$ | |
| 1.56 | $OCH_3$ | $C_6H_{13}n$ | |
| 1.57 | $OC_2H_5$ | Cyclopropyl | Smp. 35 - 56° Beisp. 1 |
| 1.58 | $OCH_3$ | Cyclohexyl | |
| 1.59 | $OC_6H_{13}$ | $CH_3$ | |
| 1.60 | $OCH_2SCH_3$ | $CH_3$ | |
| 1.61 | $OC_2H_5$ | $CH_2SCH_3$ | |
| 1.62 | $OC_2H_5$ | $C_2H_4OCH_3$ | |
| 1.63 | $OC(CH_3)_3$ | $CHClCH_3$ | |
| 1.64 | $OC_2H_5$ | $CH_2CF_3$ | |
| 1.65 | $OC_2H_5$ | $CH_2CHCl_2$ | |
| 1.66 | $OC_2H_5$ | Cyclopentyl | Öl |
| 1.67 | $OCH_3$ | Cyclobutyl | |
| 1.68 | $OCH_3$ | Cyclopentyl | |
| 1.69 | $OC_2H_5$ | Cyclobutyl | Smp. 60 - 61° |
| 1.70 | $OC_2H_5$ | Cyclopentyl | |
| 1.71 | $OCH_3$ | Cyclohexyl | |
| 1.72 | $OC_2H_5$ | Cyclohexyl | Öl |
| 1.73 | $OCH_3$ | Cyclopropyl | Smp. 60 - 64° |
| 1.74 | $OC_3H_7n$ | Cyclopropyl | |
| 1.75 | $OCH(CH_3)_2$ | Cyclopropyl | Smp. 70 - 72° |
| 1.76 | $OC_4H_9n$ | Cyclopropyl | |
| 1.77 | $OCH_2CH(CH_3)_2$ | Cyclopropyl | |
| 1.78 | $OCH(CH_3)C_2H_5$ | Cyclopropyl | |
| 1.79 | Benzyloxy | Cyclopropyl | |
| 1.80 | 4-Chlorbenzyloxy | Cyclopropyl | |
| 1.81 | 2-Chlorbenzyloxy | Cyclopropyl | |
| 1.82 | Cyclohexyloxy | Cyclopropyl | |

| | | | |
|---|---|---|---|
| 1.83 | OH | Cyclopropyl | Smp. 142 - 146° |
| 1.84 | $OCH_3$ | $C(CH_3)_3$ | |
| 1.85 | $OC_2H_5$ | $C(CH_3)_3$ | Smp. 74 - 75° |
| 1.86 | OH | $C(CH_3)_3$ | |
| 1.87 | $OC_3H_7n$ | $C(CH_3)_3$ | |
| 1.88 | $OCH(CH_3)_2$ | $C(CH_3)_3$ | |
| 1.89 | $OC_2H_5$ | $C(CH_3)_2CH_2Cl$ | |
| 1.90 | $OCH_3$ | $C(CH_3)_2CH_2Cl$ | |
| 1.91 | $OCH(CH_3)_2$ | $C(CH_3)_2CH_2Cl$ | |
| 1.92 | $OC_3H_7n$ | $C(CH_3)_2CH_2Cl$ | |
| 1.93 | OH | 1-Methylcyclopropyl | |
| 1.94 | $OCH_3$ | 1-Methylcyclopropyl | |
| 1.95 | $OC_2H_5$ | 1-Methylcyclopropyl | |
| 1.96 | $OCH(CH_3)_2$ | 1-Methylcyclopropyl | |
| 1.97 | $OC_3H_7n$ | 1-Methylcyclopropyl | |
| 1.98 | $OC_4H_9n$ | 1-Methylcyclopropyl | |
| 1.99 | OH | Cyclopentyl | Smp. 95 - 103° |
| 1.100 | OH | Cyclohexyl | Smp. 153 - 155° |
| 1.101 | OH | $CH(CH_3)C_2H_5$ | Smp. 124 - 125° |
| 1.102 | OH | 1-Methylcyclopropyl | Smp. 92 - 93° |
| 1.103 | $OC_2H_5$ | $CH(CH_3)C_2H_5$ | Öl |

**Tabelle 2** A = $NR_3R_4$

| | | | |
|---|---|---|---|
| 2.01 | $NH_2$ | $C_3H_7n$ | Smp. 167 - 169° |
| 2.02 | $NH_2$ | $C_2H_5n$ | |
| 2.03 | $N(CH_3)_2$ | $C_3H_7n$ | $n_D^{25}$ 1.5290 |
| 2.04 | $N(CH_3)_2$ | $CH(CH_3)_2$ | Smp. 60 - 62° |
| 2.05 | $NHC_4H_9$ iso | $CH(CH_3)_2$ | Smp. 126 - 128° |
| 2.06 | $NHC_4H_9$ iso | $CH_3$ | |
| 2.07 | $N(CH_2\text{-}CH=CH_2)_2$ | Cyclopropyl | |
| 2.08 | $NHCH_2\text{-}C\equiv CH$ | $CH_2OCH_3$ | |
| 2.09 | Benzylamino | $C_3H_7n$ | Wachs |
| 2.10 | 3-Nitrobenzylamino | $C_2H_4Cl$ | |
| 2.11 | 1-Methylphenyläthylamino | $C_2H_4OC_2H_5$ | |
| 2.12 | $NHC_2H_4SC_2H_5$ | $CH_2CH(CH_3)_2$ | |
| 2.13 | Piperidino | $CH(CH_3)_2$ | |
| 2.14 | Morpholino | $C_6H_{13}n$ | |
| 2.15 | $N(CH_3)_2$ | Cyclopropyl | Smp. 109 - 112° |
| 2.16 | Benzylamino | Cyclopropyl | Smp. 130 - 144° |
| 2.17 | Anilino | Cyclopropyl | |
| 2.18 | 3-Trifluormethylanilino | Cyclopropyl | |
| 2.19 | $N(CH_3)OCH_3$ | $C_3H_7n$ | Öl |
| 2.20 | $N(CH_3)OCH_3$ | $C_2H_5$ | |
| 2.21 | Anilino | $C_3H_7n$ | Smp. 123 - 127° |
| 2.22 | Anilino | $CH_3$ | |
| 2.23 | Anilino | $C_6H_{13}n$ | |
| 2.24 | Anilino | $C_2H_4CH(CH_3)_2$ | |
| 2.25 | 3-Trifluormethylanilino | $C_3H_7n$ | |
| 2.26 | 4-Methoxyanilino | $C_3H_7n$ | |
| 2.27 | 4-Chloranilino | $CH_3$ | |
| 2.28 | $N(C_2H_5)_2$ | $C_3H_7n$ | Öl |
| 2.29 | $NHCH_3$ | $C_3H_7n$ | Smp. 157 - 159° |
| 2.30 | N-Methylanilino | $C_3H_7n$ | Öl |
| 2.31 | Cyclopropylamino | $C_3H_7n$ | |
| 2.32 | $NHC_6H_{13}n$ | $C_3H_7n$ | |
| 2.33 | $NHC_2H_4SCH_3$ | $C_3H_7n$ | Wachs |
| 2.34 | $NHC_2H_4SCH_3$ | $C_2H_5$ | |
| 2.35 | $NHC_2H_4SC_3H_7i$ | $C_3H_7n$ | |
| 2.36 | Pyrrolidino | $C_3H_7n$ | Wachs |
| 2.37 | $N(CH_2CH=CH_2)_2$ | $C_3H_7n$ | Smp. 61 - 67° |
| 2.38 | $N(CH_3)_2$ | Cyclobutyl | Smp. 76 - 78° |
| 2.39 | $N(CH_3)_2$ | $C_6H_{13}n$ | |
| 2.40 | $N(CH_3)_2$ | Cyclobutyl | Smp. 76 - 78° |
| 2.41 | $NHC_3H_6SCH_3$ | $C_3H_7n$ | Wachs |
| 2.42 | $N(CH_3)_2$ | $C_2H_5$ | Smp. 89 - 91° |

| | | | |
|---|---|---|---|
| 2.43 | $N(CH_3)_2$ | $C(CH_3)_3$ | |
| 2.44 | Benzylamino | $C(CH_3)_3$ | |
| 2.45 | $N(CH_3)_2$ | $C_5H_{11}n$ | Smp. 70 - 72° |
| 2.46 | $N(CH_3)_2$ | 1-Methylcyclopropyl | Smp. 70 - 71° |
| 2.47 | Anilino | 1-Methylcyclopropyl | |
| 2.48 | 1-Methylanilino | 1-Methylcyclopropyl | |
| 2.49 | $NHC(CH_3)_3$ | 1-Methylanilino | |
| 2.50 | $N(CH_3)_2$ | $CH_3$ | Smp. 118 - 120° |

**Formulierungsbeispiele**

**Beispiel 2:**

**Formulierungsbeispiele für flüssige Wirkstoffe der Formel I (% = Gewichtsprozent)**

a) <u>Emulsionskonzentrate</u>

| Emulsionskonzentrate | a) | b) | c) |
|---|---|---|---|
| Wirkstoff | 20 % | 40 % | 50 % |
| Ca-Dodecylbenzolsulfonat | 5 % | 8 % | 5,8 % |
| Ricinusöl-polyäthylenglykoläther (36 Mol ÄO) | 5 % | - | - |
| Tributylphenol-polyäthylenglykoläther (30 mol ÄO) | - | 12 % | 4,2 % |
| Cyclohexanon | - | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

b) <u>Lösungen</u>

| Lösungen | a) | b) | c) | d) |
|---|---|---|---|---|
| Wirkstoff | 80 % | 10 % | 5 % | 95 % |
| Äthylenglykol-monomethyl-äther | 20 % | - | - | - |
| Polyäthylenglykol MC 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenze 160 - 190° C) | - | - | 94 % | - |

Die Lösungen sind zur Anwendung in Form kleinster Tropfen geeignet.

c) <u>Granulate</u>

| Granulate | a) | b) |
|---|---|---|
| Wirkstoff | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

d) <u>Stäubemittel</u>

| Stäubemittel | a) | b) |
|---|---|---|
| Wirkstoff | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

**Beispiel 3:**

**Formulierungsbeispiele für feste Wirkstoffe der Formel I (% = Gewichtsprozent)**

a) Spritzpulver

| | a) | b) |
|---|---|---|
| Wirkstoff | 20 % | 60 % |
| Na-Ligninsulfonat | 5 % | 5 % |
| Na-Laurylsulfat | 3 % | - |
| Na-Diisobutylnaphthalinsulfonat | - | 6 % |
| Octylphenolpolyäthylenglykoläther (7 - 8 Mol ÄO) | - | 2 % |
| Hochdisperse Kieselsäure | 5 % | 27 % |
| Kaolin | 67 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) Emulsions-Konzentrat

| | |
|---|---|
| Wirkstoff | 10 % |
| Octylphenolpolyäthylenglykoläther (4 - 5 Mol ÄO) | 3 % |
| Ca-Dodecylbenzolsulfonat | 3 % |
| Ricinusölpolyglykoläther (36 Mol ÄO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

c) Stäubemittel

| | a) | b) |
|---|---|---|
| Wirkstoff | 5 % | 8 % |
| Talkum | 95 % | - |
| Kaolin | - | 92 |

Man erhält anwendungsfertige Stäubemittel, indem der Wirkstoff mit dem Träger vermischt und auf einer geeigneten Mühle vermahlen wird.

d) Extruder Granulat

| | |
|---|---|
| Wirkstoff | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | |
|---|---|
| Wirkstoff | 40 % |
| Äthylenglykol | 10 % |
| Nonylphenolpolyäthylenglykoläther (15 Mol ÄO) | 6 % |
| Na-Ligninsulfonat | 10 % |
| Carboxymethylcellulose | 1 % |
| 37 %-ige wässrige Formaldehyd-Lösung | 0,2 % |
| Silikonöl in Form einer 75 %-igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

**Biologische Beispiele**

**Beispiel 4: Wuchshemmung bei Getreide**

In Kunststofftöpfen mit sterilisierter Erde werden die Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt umgerechnet 0,1 bis 2,5 kg Aktivsubstanz pro Hektar. 10 und 21 Tage nach Applikation wird das Wachstum des Getreides beurteilt. Hierbei kann festgestellt werden, dass Getreidepflanzen, die mit Wirkstoffen der Formel I behandelt worden sind, im Vergleich zu unbehandelten Kontrollpflanzen eine starke Wuchsreduktion aufweisen. Als besonders wirksam erweisen sich Verbindungen der Tabellen 1 und 2.

**Beispiel 5: Wuchshemmung bei Gräsern**

In Kunststoffschalen mit Erde-Torf-Sand-Gemisch (6 : 3 : 1) werden die Gräser Lolium perenne, Poa pratensis, Festuca ovina und Cynodon dactylon im Gewächshaus angesät und nach Bedarf bewässert. Die aufgelaufenen Gräser werden wöchentlich bis auf ca. 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet 0,1 bis 2,5 kg Aktivsubstanz pro Hektar. 10 und 21 Tage nach Applikation wird das Wachstum der Gräser beurteilt, dabei zeigt es sich, dass die erfindungsgemässen Wirkstoffe aus der Tabelle 1 eine merkliche Wuchshemmung bewirken. Besonders deutliche Wuchshemmung wird mit der Verbindung Nr. 1.1 bewirkt, so reduziert diese Verbindung das Weiterwachsen fast vollständig (Zuwachsrate 0 bis 10 %).

**Beispiel 6: Ertragssteigerung an Sojabohnen**

In Kunststoffbehältern mit einem Erde-Torf-Sand-Gemisch im Verhältnis 6 : 3 : 1 werden Sojabohnen der Sorte "Hark" angesät und in eine Klimakammer gegeben. Durch optimale Temperaturwahl, Beleuchtung, Düngerzugabe und Bewässerung entwickeln sich die Pflanzen nach ca. 5 Wochen bis zum 5 - 6 Trifola-Blattstadium. Zu diesem Zeitpunkt werden die Pflanzen mit der wässrigen Brühe eines Wirkstoffes der Formel I bis zur guten Benetzung besprüht. die Wirkstoffkonzentration beträgt bis zu 500 ppm Aktivsubstanz. Die Auswertung erfolgt ca. 5 Wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der geernteten Schoten. Als besonders wirksam erweisen sich die Verbindungen aus den Tabellen 1 und 2.

**Beispiel 7: Wuchshemmung bei Bodenbedecker-Pflanzen (Cover-Crops)**

In Kunststoffschalen mit Erde-Torf-Sand-Gemisch (1 : 1 : 1) werden Testpflanzen der Sorte Centrosema plumieri und Centrosema pubescens aus Stecklingen angezogen. Nach dem Anwurzeln werden die Pflänzchen in 9-cm-Töpfe umgetopft und nach Bedarf gewässert. Die weitere Anzucht der Pflanzen findet im Gewächshaus bei einer Tagstemperatur von 27° und eine Nachttemperatur von 21°C, bei einer mittleren Lichtdauer von 14 h (6000 Lux) und einer Luftfeuchtigkeit von 70 % statt. Die Testpflanzen werden auf eine Höhe von ca. 15 cm zurückgeschnitten und 7 Tage nach dem Zurückschneiden mit einer Spritzbrühe des Wirkstoffes (umgerechnet 0,3 bzw. 3 kg Aktivsubstanz je Hektar) besprüht. 4 Wochen nach Applikation wird das Wachstum der behandelten Pflanzen im Vergleich zu gestutzten, aber unbehandelten Kontrollpflanzen verglichen. Hierbei kann festgestellt werden, dass Verbindungen aus der Tabelle 1 eine deutliche Wuchshemmung der Bodenbedecker-Pflanzen auslösen.

**Beispiel 8: Seneszenzhemmung bei Getreidepflanzen**

Im Gewächshaus wird Sommerweizen der Sorte "Svenno" in Töpfen mit Komposterde angesät und ohne spezielle klimatische Anforderungen gezogen. Ca. 10 Tage nach dem Auflaufen werden 10 bis 12 cm lange Primärblätter abgeschnitten und einzeln in Reagenzgläser mit 10 ml einer Wirkstoffsuspension (1,25 bis 10 ppm Aktivsubstanz) gegeben. Die Reagenzgläser werden in einem klimatisierten Raum bei 23°C, 70 % relativer Luftfeuchtigkeit aufgestellt und täglich durchschnittlich 14 Stunden (10 000 Lux) bestrahlt. Die Beurteilung der Seneszenzhemmung erfolgt 7 Tage nach Einstellen der Blätter durch Vergleich des Vergilbungsgrades im Verhältnis zu noch frischen, grünen Blättern. Bei diesem Versuch kann beobachtet werden, dass diese Verbindungen eine deutliche Inhibierung der Seneszenz der Testpflanzen hervorrufen. Insbesondere die Verbindungen der Tabellen 1 und 2 hemmen das Vergilben der Blätter im Versuchszeitraum.

## Patentansprüche

1. Verfahren zur Herstellung von 4-Acyl-3,5-cyclohexandion-1-carbonsärederivate der Formel I

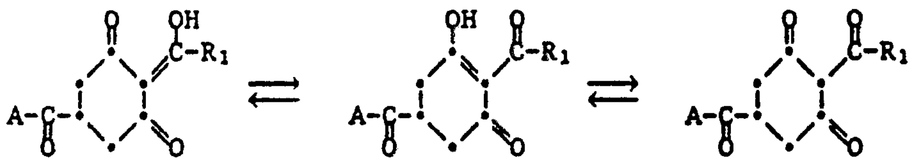 (I)

worin
A einen Rest $-OR_2$ oder $-NR_3R_4$ oder ein Metall- oder Ammoniumsalz davon,
$R_1$ $C_1$-$C_6$ Alkyl oder $C_3$-$C_6$ Cycloalkyl, unsubstituiert oder substituiert durch Halogen, $C_1$-$C_4$ Alkoxy oder $C_1$-$C_4$ Alkylthio,
$R_2$, $R_3$ und $R_4$ unabhängig voneinander je Wasserstoff; $C_1$-$C_6$ Alkyl, $C_1$-$C_4$ Halogenalkyl, $C_2$-$C_{10}$ Alkoxyalkyl, $C_2$-$C_{10}$ Alkylthioalkyl; $C_3$-$C_6$ Alkenyl, unsubstituiert oder substituiert durch Halogen, $C_1$-$C_4$ Alkoxy oder $C_1$-$C_4$ Alkylthio; $C_3$-$C_6$ Alkinyl oder Phenyl, wobei der Phenylkern unsubstituiert oder durch Halogen, $C_1$-$C_4$ Alkyl, $C_1$-$C_4$ Alkoxy, $C_1$-$C_4$ Halogenalkyl, Nitro oder Cyan substituiert ist,
$R_3$ und $R_4$ zusammen mit dem Stickstoffatom, an das sie gebunden sind, bilden auch einen 5 - 6 gliedrigen Heterocyclus, der im Ring noch ein Sauerstoff- oder Schwefelatom enthalten kann,
bedeuten, dadurch gekennzeichnet, dass man in einem inerten organischen Lösungsmittel ein reaktionsfähiges Carbonsäurederivat der Formel III

$$X-\underset{\underset{O}{\|}}{C}-R_1 \qquad (III),$$

worin X ein Halogenatom, einen Alkyl- oder Arylsulfonsäurerest oder der zum Anhydrid fehlende Molekularrest

$$-O-\underset{\underset{O}{\|}}{C}-R_1$$

bedeutet, während $R_1$ die unter der Formel I gegebene Bedeutung hat, und ein Metallcyanid zusammengibt, das Reaktionsgemisch zum sieden erhitzt und nach Abkühlen auf einen Temperaturbereich von 0°C bis Raumtemperatur, dazu nacheinander eine Lewis-Säure, die zum Carbonsäurederivat der Formel III äquimolare Menge eines 3,5-Cyclohexandion-1-carbonsäurederivates der Formel I

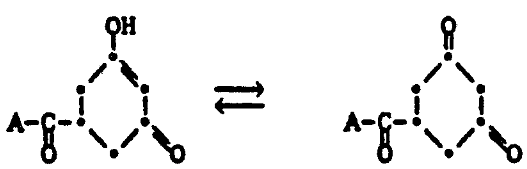 (II)

worin A die unter der Formel I gegebene Bedeutung hat, und tropfenweise eine organische Base gibt, und nach Ansäuern des Reaktionsgemisches mit einer wässrigen Säure das 4-Acyl-3,5-cyclohexandion-1-carbonsäurederivat der Formel I durch Extraktion extrahiert.

2. Verfahren gemäss Anspruch 1, dadurch gekennzeichnet, dass man als inertes organisches Lösungsmittel Acetonitril oder einen Halogenkohlenwasserstoff verwendet.

3. Verfahren gemäss Anspruch 1, dadurch gekennzeichnet, dass man als reaktionsfähiges Carbonsäurederivat der Formel III ein Halogenid oder das Chlorid verwendet.

4. Verfahren gemäss Anspruch 1, dadurch gekennzeichnet, dass man als Metallcyanid ein Alkalimetall- oder das Kupfercyanid einsetzt.

5. Verfahren gemäss Anspruch 1, dadurch gekennzeichnet, dass man das Gemisch von organischem Lösungsmittel, Carbonsäurederivat der Formel II und Metallcyanid während 1 - 25 stunden am Rückfluss erhitzt, ehe man auf einen Temperaturbereich von 0°C bis Raumtemperatur abkühlt und die Reaktion fortsetzt.

6. Verfahren gemäss Anspruch 1, dadurch gekennzeichnet, dass man als Lewis-Säure Zinkchlorid oder Aluminiumchlorid verwendet.

7. Verfahren gemäss Anspruch 1, dadurch gekennzeichnet, dass man als organische Base Diäthylamin, Trimethylamin, Triäthylamin, γ-Picolin, 4-N,N-Dimethylaminopyridin, 4-N,N-Diäthylaminopyridin, 4-Pyrrolidinopyridin, N-Methylimidazol oder 4-Äthylimidazol in das Reaktionsgemisch tropft.

8. Verfahren gemäss Anspruch 1, dadurch gekennzeichnet, dass man das Reaktionsgemisch zuletzt mit einem Salzsäure/Eis-Gemisch ansäuert.

9. Herbizides und den Pflanzenwuchs regulierendes Mittel, dadurch gekennzeichnet, dass es neben inerten Trägerstoffen und Verteilungsstoffen als Wirkstoff eine wirksame Menge eines 4-Acyl-3,5-cyclohexandion-1-carbonsäure-derivates der Formel I, Anspruch 1, enthält.

10. Neue gemäss Anspruch 1 hergestellt 4-Acyl-3,5-cyclohexandion-1-carbonsäurederivate der Formel I Anspruch 1, worin $R_1$ einen gegebenenfalls substituierten Phenylrest darstellt, während A die im Anspruch 1 gegebene Bedeutung hat.

11. 4-Cyclopropylcarbonyl-3,5-cyclohexandion-1-carbonsäure-methylester als neue, gemäss Anspruch 1 hergestellte Substanz.

12. 4-Cyclopropylcarbonyl-3,5-cyclohexandion-1-carbonsäure als neue, gemäss Anspruch 1 hergestellte Substanz.

13. 4-Trimethylacetyl-3,5-cyclohexandion-1-carbonsäure-äthylester als neue, gemäss Anspruch 1 hergestellte Substanz.

14. 4-Cyclobutylcarbonyl-3,5-cyclohexandion-1-carbonsäure-dimethylamid als neue, gemäss Anspruch 1 hergestellte Substanz.

15. 4-Butyryl-3,5-cyclohexandion-1-carbonsäuremethylthiopropylamid als neue, gemäss Anspruch 1 hergestellte Substanz.

16. 4-Butyryl-3,5-cyclohexandion-1-carbonsäure-diallylamid als neue, gemäss Anspruch 1 hergestellte Substanz.

17. 4-Butyryl-3,5-cyclohexandion-1-carbonsäure-pyrrolidonamid als neue, gemäss Anspruch 1 hergestellte Substanz.

18. 4-Propionyl-3,5-cyclohexandion-1-carbonsäure-dimethylamid als neue, gemäss Anspruch 1 hergestellte Substanz.

19. 4-Hexanoyl-3,5-cyclohexandion-1-carbonsäure-dimethylamid als neue, gemäss Anspruch 1 hergestellte Substanz.

20. 4-Cyclopropylcarbonyl-3,5-cyclohexandion-1-carbonsäureethylester als neue, gemäss Anspruch 1 hergestellte Substanz.

21. Die Verwendung der gemäss Anspruch 1 hergestellten 4-Acyl-3,5-cyclohexandion-1-carbonsäurederivate der Formel I zur Kontrolle von Unkräutern und unerwünschtem Pflanzenwuchs.

22. Die Verwendung der gemäss Anspruch 1 hergestellten 4-Acyl-3,5-cyclohexandion-1-carbonsäurederivate der Formel I zur gezielten Regulierung des Pflanzenwachstums.

## Claims

1. A process for the preparation of 4-acyl-3,5-cyclohexanedione-1-carboxylic acid derivatives of formula I

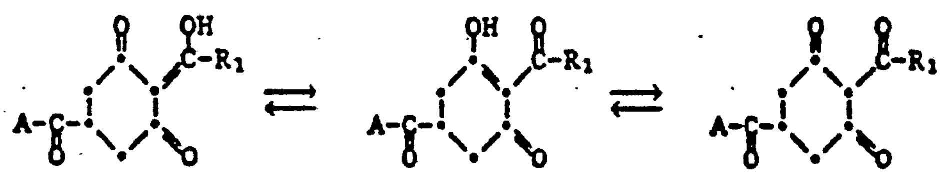

(I)

wherein

A is an $-OR_2$ or $-NR_3R_4$ radical or a metal or ammonium salt thereof,

$R_1$ is $C_1$-$C_6$alkyl or $C_3$-$C_6$cycloalkyl, each unsubstituted or substituted by halogen, $C_1$-$C_c$alkoxy or $C_1$-$C_4$-alkylthio,

$R_2$, $R_3$ and $R_4$ are each independently hydrogen; $C_1$-$C_6$alkyl, $C_1$-$C_4$haloalkyl, $C_2$-$C_{10}$alkoxyalkyl, $C_2$-$C_{10}$alkylthioalkyl; $C_3$-$C_6$alkenyl which is unsubstituted or substituted by halogen, $C_1$-$C_4$alkoxy or $C_1$-$C_4$alkylthio; $C_3$-$C_6$alkynyl; or phenyl wherein the phenyl nucleus is unsubstituted or substituted by halogen, $C_1$-$C_4$alkyl, $C_1$-

$C_4$alkoxy, $C_1$-$C_4$haloalkyl, nitro or cyano, and

$R_3$ and $R_4$, together with the nitrogen atom to which they are attached, also form a 5- or 6-membered heterocycle which may in addition contain an oxygen or sulfur atom in the ring, which process comprises combining, in an inert organic solvent, a reactive carboxylic acid derivative of formula III

$$X - C - R_1 \qquad (III)$$
||
O

wherein X is a halogen atom, an alkylsulfonic or arylsulfonic acid radical or the molecular radical

$$-O-C-R_1$$
||
O

which is necessary to form the anhydride and $R_1$ is as defined for formula I, and a metal cyanide, heating the reaction mixture to boiling point and, after cooling to a temperature in the range from 0°C to room temperature, adding in succession to said mixture a Lewis acid, an equimolar amount, with respect to the carboxylic acid derivative of formula III, of a 3,5-cyclohexanedione-1-carboxylic acid derivative of formula II

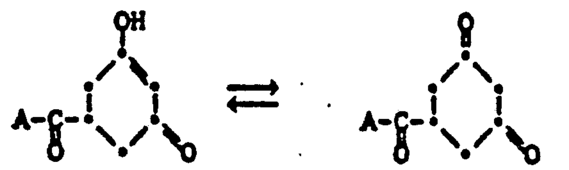

(II)

wherein A is as defined for formula I, and, dropwise, an organic base and, after acidifying the reaction mixture with an aqueous acid, isolating therefrom by extraction the 4-acyl-3,5-cyclohexanedione-1-carboxylic acid derivative of formula I.

2. A process according to claim 1, wherein the inert organic solvent is acetonitrile or a halogenated hydrocarbon.

3. A process according to claim 1, wherein the reactive carboxylic acid derivative of formula III is a halide or the chloride.

4. A process according to claim 1, wherein the metal cyanide is an alkali metal cyanide or copper cyanide.

5. A process according to claim 1, which comprises heating the mixture of organic solvent, carboxylic acid derivative of formula II and metal cyanide for 1 to 25 hours under reflux before cooling to a temperature in the range from 0°C to room temperature and continuing the reaction.

6. A process according to claim 1, wherein the Lewis acid is zinc chloride or aluminium chloride.

7. A process according to claim 1, wherein there is added dropwise as organic base to the reaction mixture diethylamine, trimethylamine, triethylamine, γ-picoline, 4-N,N-dimethylaminopyridine, 4-N,N-diethylaminopyridine, 4-pyrrolidinopyridine, N-methylimidazole or 4-ethylimidazole.

8. A process according to claim 1 which comprises ultimately acidifying the reaction mixture with a mixture of hydrochloric acid and ice.

9. A herbicidal and plant growth-regulating composition which contains, as active ingredient, an effective amount of a 4-acyl-3,5-cyclohexanedione-1-carboxylic acid derivative of formula I, claim 1, together with inert carriers and dispersing agents.

10. Novel 4-acyl-3,5-cyclohexanedione-1-carboxylic acid derivatives of formula 1, claim 1, prepared according to claim 1, wherein $R_1$ is an unsubstituted or substituted phenyl radical and A is as defined in claim 1.

11. 4-Cyclopropylcarbonyl-3,5-cyclohexanedione-1-carboxylic acid methyl ester as novel substance prepared according to claim 1.

12. 4-Cyclopropylcarbonyl-3,5-cyclohexanedione-1-carboxylic acid as novel substance prepared according to claim 1.

13. 4-Trimethylacetyl-3,5-cyclohexanedione-1-carboxylic acid ethyl ester as novel substance prepared according to claim 1.

14. 4-Cyclobutylcarbonyl-3,5-cyclohexanedione-1-carboxylic acid dimethyl amide as novel substance prepared according to claim 1.

15. 4-Butyryl-3,5-cyclohexanedione-1-carboxylic acid methylthiopropyl amide as novel substance prepared according to claim 1.

16. 4-Butyryl-3,5-cyclohexanedione-1-carboxylic acid diallyl amide as novel substance prepared according to claim 1.

17. 4-Butyryl-3,5-cyclohexanedione-1-carboxylic acid pyrrolidone amide as novel substance prepared according to claim 1.

18. 4-Propionyl-3,5-cyclohexanedione-1-carboxylic acid dimethyl amide as novel substance prepared according to claim 1.

19. 4-Hexanoyl-3,5-cyclohexanedione-1-carboxylic acid dimethyl amide as novel substance prepared according to claim 1.

20. 4-Cyclopropylcarbonyl-3,5-cyclohexanedione-1-carboxylic acid ethyl ester as novel substance prepared according to claim 1.

21. The use of the 4-acyl-3,5-cyclohexanedione-1-carboxylic acid derivatives of formula 1 prepared according to claim 1 for controlling weeds and undesired plant growth.

22. The use of 4-acyl-3,5-cyclohexanedione-1-carboxylic acid derivatives of formula 1 prepared according to claim 1 for the controlled regulation of plant growth.

**Revendications**

1. Procédé de préparation de dérivés d'acides 4-acyl-3,5-cyclohexane-dione-1-carboxyliques de formule I

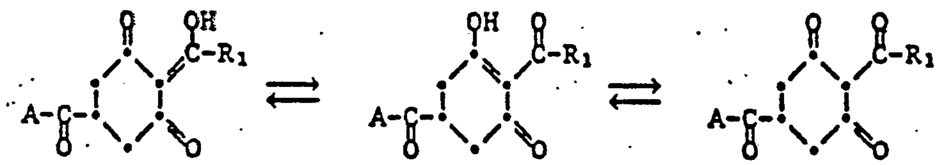
(I)

dans laquelle

A représente un groupe $-OR_2$ ou $-NR_3R_4$ ou un sel métallique <u>ou</u> d'ammonium d'un tel groupe,

$R_1$ représente un groupe alkyle en $C_1$-$C_6$ ou cycloalkyle en $C_3$-$C_6$, non substitué ou substitué par des halogènes, des groupes alcoxy en $C_1$-$C_4$ ou alkylthio en $C_1$-$C_4$,

$R_2$, $R_3$ et $R_4$ représentent chacun, indépendamment les uns des autres, l'hydrogène; un groupe alkyle en $C_1$-$C_6$, halogénoalkyle en $C_1$-$C_4$, alcoxyalkyle en $C_2$-$C_{10}$, alkylthioalkyle en $C_2$-$C_{10}$; un groupe alcényle en $C_3$-$C_6$ non substitué ou substitué par des halogènes, des groupes alcoxy en $C_1$-$C_4$ ou alkylthio en $C_1$-$C_4$; un groupe alcynyle en $C_3$-$C_6$ ou un groupe phényle dans lequel le noyau phényle peut être non substitué ou substitué par des halogènes, des groupes alkyle en $C_1$-$C_4$, alcoxy en $C_1$-$C_4$, halogénoalkyle en $C_1$-$C_4$, nitro ou cyano,

$R_3$ et $R_4$ peuvent également former avec l'atome d'azote auquel ils sont reliés un hétérocycle à 5 ou 6 chaînons qui peut encore contenir dans le cycle un atome d'oxygène ou de soufre,

caractérisé en ce que l'on met en contact dans un solvant organique inerte un dérivé réactif d'acide carboxylique de formule III

$$X-\underset{\underset{O}{\|}}{C}-R_1 \qquad (III)$$

dans laquelle X représente un atome d'halogène, un radical d'acide alkyl- ou aryl-sulfonique ou le radical moléculaire manquant à l'anhydride

$$-O-\underset{\underset{O}{\|}}{C}-R_1,$$

$R_1$ ayant les significations indiquées en référence à la formule I, et un cyanure métallique, on chauffe le mélange de réaction à l'ébullition et après refroidissement, on ajoute successivement, dans un intervalle de température allant de 0°C jusqu'à la température ambiante, un acide de Lewis, un dérivé d'acide 3,5-cyclohexanedione-1-carboxylique de formule II

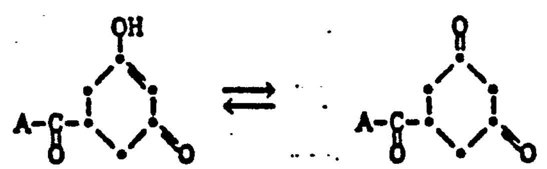
(II)

dans laquelle A a les significations indiquées en référence à la formule I, en quantité équimoléculaire par rapport au dérivé d'acide carboxylique de formule III; et goutte à goutte, une base organique, et, après acidification à l'aide d'un acide aqueux, on isole par extraction le dérivé d'acide 4-acyl-3,5-cyclohexanedione-1-carboxylique de formule I.

2. Procédé selon la revendication 1, caractérisé en ce que l'on utilise en tant que solvant organique inerte l'acétonitrile ou un hydrocarbure halogéné.

3. Procédé selon la revendication 1, caractérisé en ce que l'on utilise en tant que dérivé réactif d'acide carboxylique de formule III un halogénure ou le chlorure.

4. Procédé selon la revendication 1, caractérisé en ce que l'on utilise en tant que cyanure métallique un cyanure de métal alcalin ou le cyanure de cuivre.

5. Procédé selon la revendication 1, caractérisé en ce que l'on chauffe au reflux le mélange du solvant organique, du dérivé d'acide carboxylique de formule II et du cyanure métallique pendant 1 à 25 heures, on refroidit dans un intervalle de température allant de 0°C jusqu'à la température ambiante et on poursuit la réaction.

6. Procédé selon la revendication 1, caractérisé en ce que l'on utilise en tant qu'acide de Lewis le chlorure de zinc ou le chlorure d'aluminium.

7. Procédé selon la revendication 1, caracterisé en ce que l'on utilise en tant que base organique, qu'on introduit goutte à goutte dans le mélange de réaction, la diéthylamine, la triméthylamine, la triéthylamine, la gamma-picoline, la 4-N,N-diméthylaminopyridine, la 4-N,N-diéthylaminopyridine, la 4-pyrrolidinopyridine, le 4-méthylimidazole ou le 4-éthylimidazole.

8. Procédé selon la revendication 1, caractérisé en ce que l'on acidifie finalement le mélange de réaction à l'aide d'un mélange acide chlorhydrique/glace.

9. Produit herbicide et régulateur de croissance des végétaux, caractérisé en ce qu'il contient en tant que substance active, en quantité efficace, avec des véhicules inertes et des diluants, un dérivé d'acide 4-acyl-3,5-cyclohexane-dione-1-carboxylique de formule I, revendication 1.

10. Nouveaux dérivés d'acides 4-acyl-3,5-cyclohexane-dione-1-carboxyliques de formule I, revendication 1, préparés selon revendication 1, dans lesquels $R_1$ représente un groupe phényle éventuellement substitué et A a les significations indiquées dans la revendication 1.

11. Le 4-cyclopropylcarbonyl-3,5-cyclohexanedione-1-carboxylate de méthyle en tant que nouvelle substance préparée selon la revendication 1.

12. L'acide 4-cyclopropylcarbonyl-3,5-cyclohexane-dione-1-carboxylique en tant que nouvelle substance préparée selon la revendication 1.

13. Le 4-triméthylacétyl-3,5-cyclohexanedione-1-carboxylate d'éthyle en tant que nouvelle substance préparée selon la revendication 1.

14. Le diméthylamide de l'acide 4-cyclobutylcarbonyl-3,5-cyclohexane-dione-1-carboxylique en tant que nouvelle substance préparée selon la revendication 1.

15. Le méthylthiopropylamide de l'acide 4-butyryl-3,5-cyclohexane-dione-1-carboxylique en tant que nouvelle substance préparée selon la revendication 1.

16. Le diallylamide de l'acide 4-butyryl-3,5-cyclohexane-dione-1-carboxylique en tant que nouvelle substance préparée selon la revendication 1.

17. Le pyrrolidonamide de l'acide 4-butyryl-3,5-cyclohexane-dione-1-carboxylique en tant que nouvelle substance préparée selon la revendication 1.

18. Le diméthylamide de l'acide 4-propionyl-3,5-cyclohexane-dione-1-carboxylique en tant que nouvelle substance préparée selon la revendication 1.

19. Le diméthylamide de l'acide 4-hexanoyl-3,5-cyclohexane-dione-1-carboxylique en tant que nouvelle substance préparée selon la revendication 1.

20. Le 4-cyclopropylcarbonyl-3,5-cyclohexane-dione-1-carboxylate d'éthyle en tant que nouvelle substance préparée selon la revendication 1.

21. L'utilisation des dérivés d'acides 4-acyl-3,5-cyclohexane-dione-1-carboxyliques de formule I préparés selon la revendication 1 dans la lutte contre les végétaux adventices et les croissances de végétaux indésirables.

22. L'utilisation des dérivés d'acides 4-acyl-3,5-cyclohexane-dione-1-carboxyliques de formule I préparés selon la revendication 1 pour la régulation intentionnelle de la croissance des végétaux.